# EUROPEAN PATENT SPECIFICATION

(11) **EP 1 992 227 B1**
(45) Date of publication and mention of the grant of the patent: **23.09.2015**
(21) Application number: 08251402.7
(22) Date of filing: 11.04.2008
(51) Int. Cl.: A01N 43/80, A01N 25/02, A01N 25/22, A01N 33/08, A01N 43/78, A01N 47/12, A01N 59/12

(54) **Stabilized fluids**
Stabilisierte Flüssigkeiten
Fluides stabilisés

(30) Priority: 08.05.2007 US 928230 P
(43) Date of publication of application: 19.11.2008
(73) Proprietor: Rohm and Haas Company, Philadelphia, PA 19106-2399 (US)
(72) Inventor: Williams, Terry Michael, Lower Gwynedd Pennsylvania 19002 (US)
(74) Representative: Kent, Venetia Katherine

(56) References cited:
- EP-A- 0 745 326
- US-A- 5 210 094
- US-A- 5 910 503

## Description

### BACKGROUND:

A variety of useful fluids contain one or more 3-isothiazolone and contain a relatively high concentration of one or more alkanolamine. Some 3-isothiazolones are not stable during storage in some of such fluids, and it is desired to also include a stabilizer in the fluid. One example of such a fluid is a metalworking fluid concentrate, which is a fluid that, after dilution by a factor of at least 10, is useful as a metalworking fluid.

US Patent No. 5,210,094 discloses metalworking fluids and metalworking fluid concentrates that contain 3-isothiazolones and a sulfur-containing compound.

It is desired to provide fluids that contain one or more 3-isothiazolone and that contain a relatively high concentration of certain alkanolamines, in which the one or more 3-isothiazolone is stable on storage.

### STATEMENT OF THE INVENTION:

The present invention, in its various aspects, is as set out in the accompanying claims.

In one aspect of the present invention, there is provided a fluid composition comprising
(a) one or more non-chlorinated 3-isothiazolone selected from methylisothiazolone and octylisothiazolone,
(b) 0.7% to 10% by weight, based on the weight of said fluid composition, one or more primary alkanolamine,
(c) 1% to 30% by weight, based on the weight of said fluid composition, one or more tertiary alkanolamine,
(d) one or more stabilizer selected from the group consisting of iodic acid, periodic acid, iodate salts, periodate salts, iodopropynylbutylcarbamate, mercaptobenzothiazole, and mixtures thereof,
wherein the weight ratio of said stabilizer to said non-chlorinated 3-isothiazolone is from 0.2:1 to 5:1.

### DETAILED DESCRIPTION:

A fluid composition is a composition that is liquid from 15°C to 60°C or possibly over a broader temperature range.

A 3-isothiazolone is a compound of the formula 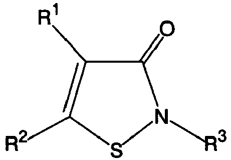 wherein R¹, R², and R³ is each independently a hydrogen or a halogen or a substituted or unsubstituted organic radical. R¹ and R² may or may not be connected to each other to form a ring structure. If any one or more of R¹, R², and R³ has one or more chlorine atom, the compound is known as a chlorinated 3-isothiazolone. If any one or more of R¹, R², and R³ has one or more halogen atom, the compound is known as a halogenated 3-isothiazolone. If none of R¹, R², and R³ has any chlorine atoms, the compound is known as a non-chlorinated 3-isothiazolone. If none of R¹, R², and R³ has any halogen atoms, the compound is known as a non-halogenated 3-isothiazolone.

The non-chlorinated 3-isothiazolone suitable for use in the present invention are methylisothiazolone and octylisothiazolone.

In some embodiments, the fluid composition contains any one of or any mixture of the non-chlorinated 3-isothiazolones defined herein above. It is contemplated that the fluid composition of the present invention contains at least one 3-isothiazolone that has no chlorine atom., In some embodiments, the fluid composition contains no 3-isothiazolone that has a chlorine atom.

In some embodiments, the fluid composition contains one or more of 2-methyl-3-isothiazolone or 2-n-octyl-3-isothiazolone or a mixture thereof. Independently, in some embodiments, the fluid composition contains no halogenated 3-isothiazolones.

Independently, in some embodiments, the fluid composition contains no 3-isothiazolone other than 2-methyl-3-isothiazolone and 2-n-octyl-3-isothiazolone.

Independent of the type of non-chlorinated 3-isothiazolone used, the amount of non-chlorinated 3-isothiazolone may vary widely. It is contemplated that a specific fluid composition, used under specific conditions, will have greater or lesser tendency to form biological growth, and thus a larger or smaller amount of non-chlorinated 3-isothiazolone, which is generally considered to be an effective biocide, will be used. In some embodiments, the amount of non-chlorinated 3-isothiazolone, by weight based on the weight of fluid composition, is 200 ppm or more; or 500 ppm or more, or 900 ppm or more. Independently, in some embodiments, the amount of non-chlorinated 3-isothiazolone, by weight based on the weight of fluid composition, is 20,000 ppm or less; or 10,000 ppm or less, or 5,000 ppm or less.

An alkanol group is a group with the structure HO-R⁷-, wherein R⁷ is an alkyl or alkoxyalkyl group. R⁷ may be straight, branched, cyclic, or a combination thereof. Some suitable alkanol groups are, for example, -CH₂OH, -CH₂CH₂OH, -CH₂CH₂CH₂OH, -C(CH₃)₂CH₂OH, -CH₂CH₂-O-CH₂CH₂OH,

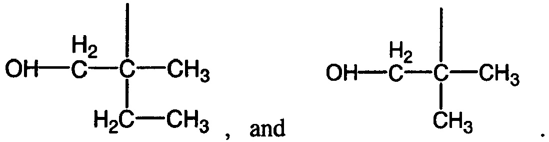

In some embodiments, alkanol groups include -CH₂CH₂OH, -C(CH₃)₂CH₂OH, and -CH₂CH₂-O-CH₂CH₂OH.

An alkanolamine is a compound with the structure 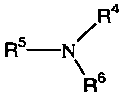 wherein R⁴ an alkanol group, and wherein R⁵ and R⁶ is each independently a hydrogen or a substituted or unsubstituted organic radical.

A primary alkanolamine is an alkanolamine in which R⁵ and R⁶ is each a hydrogen. In some embodiments, a primary alkanolamine is used in which the alkanol group has four or five carbon atoms. In some embodiments, a primary alkanolamine is used in which the alkanol group has four carbon atoms. Independently, some embodiments, a primary alkanolamine is used in which the alkanol group is -CH₂CH₂OH, -C(CH₃)₂CH₂OH, -CH₂CH₂-O-CH₂CH₂OH, or 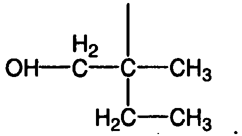 Independently, in some embodiments, a primary alkanolamine is used in which the alkanol group is -C(CH₃)₂CH₂OH or -CH₂CH₂-O-CH₂CH₂OH. In some embodiments, no primary alkanolamine is used that has an alkanol group other than -C(CH₃)₂CH₂OH or -CH₂CH₂-O-CH₂CH₂OH.

A tertiary alkanolamine is an alkanolamine in which neither R⁵ nor R⁶ is a hydrogen. Each of R⁵ and R⁶ may, for example, be a substituted or unsubstituted alkyl group (straight, branched, cyclic, or a combination thereof), a substituted or unsubstituted aryl group, or a combination thereof. In some embodiments, at least one of R⁵ and R⁶ is an unsubstituted alkyl group or an alkanol group. In some embodiments, both of R⁵ and R⁶ are selected from unsubstituted alkyl groups, alkanol groups, and mixtures thereof. In some embodiments, no tertiary alkanolamine is used other than those in which both of R⁵ and R⁶ are selected from unsubstituted alkyl groups, alkanol groups, and mixtures thereof. Independently, in some embodiments, at least one of R⁵ and R⁶ is an alkanol group.

Among embodiments in which at least one of R⁵ and R⁶ is an unsubstituted alkyl group, some suitable unsubstituted alkyl groups are, for example, C₁ to C₄ alkyl groups that are straight or branched. In some embodiments, at least one of R⁵ and R⁶ is a methyl group. Independently, among embodiments in which at least one of R⁵ and R⁶ is an alkanol group, suitable alkanol groups include, for example, -CH₂OH, -CH₂CH₂OH, -CH₂CH₂CH₂OH, and 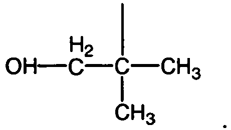 In some embodiments, at least one of R⁵ and R⁶ is -CH₂CH₂OH.

Some suitable tertiary alkanolamines include, for example, triethanolamine, bis(hydroxyethyl)methylamine (also called N-methyldiethanolamine), 2-dimethylamino-2-methylpropanol, and mixtures thereof. In some embodiments, the tertiary alkanolamine includes triethanolamine, bis(hydroxyethyl)methylamine, or mixtures thereof. In some embodiments, no tertiary alkanolamine is used other than triethanolamine or bis(hydroxyethyl)methylamine.

The fluid compositions of the present invention contain one or more primary alkanolamine in an amount chosen so that the amount of all primary alkanolamines is 0.7% or more by weight, based on the weight of the fluid composition. In some embodiments, the amount of all primary alkanolamines is, by weight based on the weight of the fluid composition, 1.5% or more, or 3% or more.

Independently, the fluid compositions of the present invention contain one or more primary alkanolamine in an amount chosen so that the amount of all primary alkanolamines is 10% or less by weight, based on the weight of the fluid composition. In some embodiments, the amount of all primary alkanolamines is, by weight based on the weight of the fluid composition, 7% or less, or 5% or less.

The fluid compositions of the present invention contain one or more tertiary alkanolamine in an amount chosen so that the amount of all tertiary alkanolamines is 2% or more by weight, based on the weight of the fluid composition. In some embodiments, the amount of all tertiary alkanolamines is, by weight based on the weight of the fluid composition, 4% or more, or 8% or more.

Independently, the fluid compositions of the present invention contain one or more tertiary alkanolamine in an amount chosen so that the amount of all tertiary alkanolamines is 30% or less by weight, based on the weight of the fluid composition. In some embodiments, the amount of all tertiary alkanolamines is, by weight based on the weight of the fluid composition, 20% or less, or 15% or less.

In some embodiments, the fluid composition of the present invention contains no secondary alkanolamine. A secondary alkanolamine is an alkanolamine in which exactly one of R⁵ and R⁶ is a hydrogen.

The fluid composition of the present invention contains one or more stabilizer selected from iodine-containing stabilizers, mercaptobenzothiazole, and mixtures thereof. Iodine-containing stabilizers are compounds that contain at least one iodine atom per molecule and that are effective at stabilizing non-chlorinated 3-isothiazolones when used in fluid compositions of the present invention. The iodine-containing stabilizers are selected from the group consisting of iodic acid, periodic acid, iodate salts, periodate salts, and iodopropynylbutylcarbamate. Iodate salts include, for example, alkali metal salts. One suitable iodate salt is potassium iodate. Periodate salts include, for example, alkali metal salts. One suitable periodate salt is potassium periodate.

In some embodiments, the fluid composition contains one or more iodine-containing stabilizer. Independently, in some embodiments, the fluid composition contains one or more stabilizer selected from iodic acid, iodate salts, periodic acid, periodate salts, and mixtures thereof. Independently, in some embodiments, the fluid composition contains one or more stabilizer selected from iodic acid, potassium iodate, iodopropynylbutylcarbamate, mercaptobenzothiazole, and mixtures thereof. Independently, in some embodiments, the fluid composition contains one or more stabilizer selected from iodic acid, potassium iodate, iodopropynylbutylcarbamate, and mixtures thereof. Independently, in embodiments, the fluid composition contains one or more stabilizer selected from iodic acid, potassium iodate, and mixtures thereof.

Independent of the type of stabilizer that is used, the amount of stabilizer is chosen according to the weight ratio of stabilizer to non-chlorinated 3-isothiazolone. In the practice of the present invention, that weight ratio is from 0.2:1 to 5:1. In some embodiments, that weight ratio is 0.5 or larger, or 0.75 or larger, or 0.9 or larger. Independently, in some embodiments, that weight ratio is 2 or lower, or 1.5 or lower. As used herein, when a ratio is said to be "X or larger," it is meant that the ratio is Y:1, where Y is equal to or greater than X. Similarly, when a ratio is said to be "W or lower," it is meant that the ratio is Z:1, where Z is equal to or less than W.

In some embodiments, the fluid composition of the present invention contains no pyridine-N-oxide, pyridine, 2-pyrrolidone, 1-methyl-2-pyrrolidone, s-triazine, or dimethyl oxime. In some embodiments, the fluid composition contains no nitrogen-based heterocyclic compounds. In some embodiments, the fluid composition contains no nitrogen-containing compounds that are not alkanolamines and that are capable of reversibly forming an adduct with any of the non-chlorinated 3-isothiazolones described herein above.

In some embodiments, the fluid composition of the present invention contains no 2-mercaptopyridine, 4-mercaptopyridine, sodium salt of 2-mercaptopyridine-N-oxide, 2-mercaptobenzothiazole, 4-methyl-4-H-1,2,4-triazole-3-thiol, 2-methylthiobenzothiazole, 2-thiohydantoin, methylenebisthiocyante, L-cystin, or 4-R(thiazolidene-thione-4-carbonic acid). In some embodiments, the fluid composition contains no nitrogen-based heterocyclic thiols. In some embodiments, the fluid composition contains no compounds having a sulfur atom attached to a nitrogen-containing aromatic ring. In some embodiments, the fluid composition contains no sulfur-containing compound or salt thereof capable of reversibly forming an adduct with any of the non-chlorinated 3-isothiazolones described herein above.

In some embodiments, the fluid composition of the present invention contains no aromatic disulfide.

The fluid compositions of the present invention may be used for any purpose. For example, the fluid compositions, optionally diluted and/or optionally mixed with additional ingredients, may form preparations that are useful for any of a wide variety of purposes. In some cases, such preparations are subject to contamination by bacteria, fungi, yeast, or algae, and it is contemplated that non-chlorinated 3-isothiazolone may provide useful biocide properties. Independently, in some cases, such preparations may be useful as one or more of metalworking fluid, industrial process water, laundry rinse water, coatings, adhesives, lubricants, process additives, cosmetics, caulks, and personal care products.

In some embodiments, a fluid composition of the present invention further contains one or more metalworking additive. Metalworking additives include, for example, fatty acids, surfactants, soluble oils, emulsifiable oils, and mixtures thereof. In some embodiments, a fluid composition of the present invention contains one or more surfactant, one or more fatty acid, or a mixture thereof.

Independently, in some embodiments, a fluid composition of the present invention is suitable as a metalworking fluid concentrate. That is, diluting the fluid composition of the present invention by a factor of at least 10 produces a preparation that is suitable as a metalworking fluid, or in some cases, a preparation that becomes suitable as a metalworking fluid after addition of additional ingredients. In some of such embodiments, the fluid composition of the present invention, prior to dilution, is not suitable as a metalworking fluid.

As used herein, diluting the fluid composition by a factor of F means mixing the fluid composition with a solvent, where the ratio of weight of solvent to weight of fluid composition is F.

In some embodiments, the fluid composition of the present invention is used to produce a preparation suitable as a metalworking fluid by dilution with an aqueous solvent. An aqueous solvent is a solvent that contains 50% or more water by weight, based on the weight of the solvent. In some embodiments, an aqueous solvent is used that has water, by weight based on the weight of the solvent, of 75% or more, or 90% or more, or 95% or more.

In some embodiments, a preparation suitable as a metalworking fluid is made by diluting a fluid composition of the present invention by a factor of 15 or more. Independently, in some embodiments, a preparation suitable as a metalworking fluid is made by diluting a fluid composition of the present invention by a factor of 50 or less, or 25 or less.

### EXAMPLES

In the following examples, the non-chlorinated 3-isothiazolones were methylisothiazolone (MIT) and octylisothiazolone (OIT). The primary alkanolamines used were monoethanolamine (MEA), 2-amino-2-methyl-1-propanol (AMP), monoisopropanolamine (MIPA), and 2-(2-aminoethoxy)-ethanol (AEE). Secondary alkanolamine used was 2-butylaminoethanol (BAE). Tertiary alkanolamines were triethanolamine (TEA) and bis(hydroxyethyl)methylamine (BHEMA).

### Test Formulation

The formulation used for these tests was prepared in 2 stages. The following amounts were used to prepare 100 grams of the formulation. For stage one, the following was added: distilled water, 2.7 g; tertiary or secondary amine, 10.1 g; primary amine, 2.6 g; boric acid, 2.0 g; Corfree™ M1, 1.2 g; pelargonic acid, 0.2 g; caprylic acid, 0.2 g; citric acid, 0.1 g; and glycerin, 0.2g. Each of the above components was added individually and in order with heating (50°C) and mixing. Each ingredient was allowed to thoroughly dissolve before adding the next component. Heating of the mixture was discontinued after all ingredients were added. The following ingredients were added to the stage one mixture: distilled water, 65.7 g; primary amine, 1.0 g; Pluronic™ 25R (100% polyoxypropylene-polyoxyethylene block copolymer), 10.0 g; caprylic acid, 2.0 g; sodium tolyltriazole - 50% solution, 1.0 g; and biocide, 0.3-0.6 g. The ingredients were added individually and in the above order at room temperature with mixing. Each ingredient was allowed to thoroughly dissolve before adding the next component.

It is contemplated that this formulation would be suitable as a metalworking fluid if it were diluted with water by a factor of 20.

Each formulation received an addition of one of the following stabilizers, at a 1:1 ratio to the biocide active ingredient to stabilizer. Stabilizers tested were potassium iodate, iodic acid, iodopropynylbutylcarbamate (IPBC) and mercaptobenzothiazole (MBZ). A sample without stabilizer served as a control. Biocide additions (by weight of active ingredient) to formulations were as follows: 2,000 ppm MIT, 1,000 ppm OIT, and 4,000ppm BIT. The biocides used in this study were Kordek™ LX 5000 (50% MIT), Kathon™ 893 MW (45% OIT), and Rocima™ BT 2S (19% BIT).

Samples were aged at 50°C for 30 days to determine the percent biocide remaining over time. The 3-isothiazolone content was measured by high pressure liquid chromatography (HPLC) at time zero (initial, prior to aging) and after 30 days. The % remaining of the biocides are reported for each amine combination and in the presence or absence of 4 stabilizers. The initial pH of the various formulations was pH 10-11.

Corfree™ M1 is a registered trademark of INVISTA, and is a mixture containing dibasic acids, primarily dodecanedioic acid (38-49%) and undecanedioic acid (3 1-38%), sebacic acid (5-7%), other dibasic acids (9-19%), other organics (7-11%), nitrogen (0.5%), and water (0.3%).

Pluronic™ is a registered trademark of BASF Corporation.

Kordek, Kathon, and Rocima are registered trademarks of the Rohm and Haas Company.

### Example 1: Results for MIT

| | | | % MIT Remaining | | | | |
|---|---|---|---|---|---|---|---|
| Sample # | Primary Amine | Secondary or Tertiary Amine | No Stabilizer | Potassium Iodate | Iodic Acid | IPBC | MBZ |
| 1 | MEA | TEA | 4 | 68 | 67 | 3 | 5 |
| 2 | AEE | TEA | 3 | 92 | 92 | 0 | 3 |
| 3 | MIPA | TEA | 3 | 86 | 95 | 83 | 5 |
| 4 | AMP | TEA | 6 | 93 | 98 | 85 | 0 |
| 5 | MEA | BHEMA | 8 | 72 | 7 | 6 | 8 |
| 6 | AEE | BHEMA | 10 | 71 | 78 | 7 | 9 |
| 7 | MIPA | BHEMA | 16 | 97 | 98 | 5 | 8 |
| 8 | AMP | BHEMA | 12 | 100 | 100 | 100 | 100 |
| 9(Comparative) | MEA | BAE | 5 | 1 | 2 | 3 | 5 |
| 10(Comparative) | AEE | BAE | 4 | 2 | 1 | 3 | 5 |
| 11(Comparative) | MIPA | BAE | 2 | 2 | 0 | 2 | 5 |
| 12(Comparative) | AMP | BAE | 2 | 2 | 1 | 2 | 4 |

In the absence of a stabilizer, MIT degraded significantly. None of the stabilizers were effective (no increased stability of MIT) with any of the combinations using BAE as the secondary amine. Combinations with TEA or BHEMA as the tertiary amine provided greatly improved stability of the MIT (from 67 to 100% remaining) with one or more of the stabilizers. The AMP/BHEMA amine combination (#8) was most effective with no measurable loss of biocide with all stabilizers tested (100% remaining). Combinations #3 (MIPA/TEA) and #4 (AMP/TEA) also showed excellent stability with three of the four stabilizers. Overall, iodate and iodic acid demonstrated the best improvement in stability of MIT with 8 amine combinations showing greater than 60% of the biocide remaining. IPBC and MBZ were effective stabilizers with three and one amine combination, respectively.

### Example 2: Results for OIT

| | | | OIT Remaining | | | | |
|---|---|---|---|---|---|---|---|
| Sample # | Primary Amine | Secondary or Tertiary Amine | No Stabilizer | Potassium Iodate | Iodic Acid | IPBC | MBZ |
| 1 | MEA | TEA | 0 | 87 | 80 | 0 | 0 |
| 2 | AEE | TEA | 0 | 85 | 78 | 15 | 17 |
| 3 | MIPA | TEA | 13 | 96 | 78 | 0 | 0 |
| 4 | AMP | TEA | 0 | 77 | 83 | 83 | 80 |
| 5 | MEA | BHEMA | 0 | 57 | 68 | 0 | 0 |
| 6 | AEE | BHEMA | 13 | 45 | 49 | 12 | 0 |
| 7 | MIPA | BHEMA | 0 | 72 | 80 | 0 | 0 |
| 8 | AMP | BHEMA | 0 | 94 | 100 | 85 | 96 |
| 9 (Comparative) | MEA | BAE | 0 | 5 | 0 | 0 | 0 |
| 10(Comparative) | AEE | BAE | 0 | 0 | 0 | 0 | 0 |
| 11 (Comparative) | MIPA | BAE | 0 | 0 | 0 | 0 | 0 |
| 12(Comparative) | AMP | BAE | 0 | 0 | 0 | 0 | 0 |

In the absence of a stabilizer, OIT degraded completely in 10 fluids and only 13 % remaining in two combinations. None of the stabilizers were effective (no increased stability of OIT) with any of the combinations using BAE as the secondary amine. Combinations with TEA or BHEMA as the tertiary amine provided greatly improved stability of the OIT (from 45 to 100% remaining) with one or more of the stabilizers. Amine combinations #8 (AMP/BHEMA) and #4 (AMP/TEA) were most effective with 77-100% of the biocide remaining with the four stabilizers tested. Iodate and iodic acid demonstrated the best overall improvement in stability of OIT with 8 amine combinations showing greater than 60% of the biocide remaining. IPBC and MBZ were effective stabilizers with two amine combinations.

## Claims

1. A fluid composition comprising
(a) one or more non-chlorinated 3-isothiazolone selected from methylisothiazolone and octylisothiazolone,
(b) 0.7% to 10% by weight, based on the weight of said fluid composition, one or more primary alkanolamine,
(c) 2% to 30% by weight, based on the weight of said fluid composition, one or more tertiary alkanolamine,
(d) one or more stabilizer selected from the group consisting of iodic acid, periodic acid, iodate salts, periodate salts, iodopropynylbutylcarbamate, mercaptobenzothiazole, and mixtures thereof,
wherein the weight ratio of said stabilizer to said non-chlorinated 3-isothiazolone is from 0.2:1 to 5:1.

2. A dilute fluid composition formed by a process comprising diluting the fluid composition of claim 1 by a factor of 10 to 50, wherein said dilute fluid composition is suitable as a metalworking fluid.

## Patentansprüche

1. Eine Fluidzusammensetzung, die Folgendes beinhaltet:
(a) ein oder mehrere nichtchlorierte 3-lsothiazolone, ausgewählt aus Methylisothiazolon und Octylisothiazolon,
(b) zu 0,7 Gew.-% bis 10 Gew.-%, bezogen auf das Gewicht der Fluidzusammensetzung, ein oder mehrere primäre Alkanolamine,
(c) zu 2 Gew.-% bis 30 Gew.-%, bezogen auf das Gewicht der Fluidzusammensetzung, ein oder mehrere tertiäre Alkanolamine,
(d) ein oder mehrere Stabilisatoren, ausgewählt aus der Gruppe, bestehend aus lodsäure, Periodsäure, lodatsalzen, Periodatsalzen, Iodpropinylbutylcarbamat, Mercaptobenzothiazol und Mischungen davon,
wobei das Gewichtsverhältnis des Stabilisators zu dem nichtchlorierten 3-Isothiazolon von 0,2 : 1 bis 5 : 1 beträgt.

2. Eine verdünnte Fluidzusammensetzung, gebildet durch ein Verfahren, das das Verdünnen der Fluidzusammensetzung gemäß Anspruch 1 um einen Faktor von 10 bis 50 beinhaltet, wobei die verdünnte Fluidzusammensetzung als Metallbearbeitungsfluid geeignet ist.

## Revendications

1. Une composition de fluide comprenant
(a) une 3-isothiazolone non chlorée ou plus sélectionnée parmi une méthylisothiazolone et une octylisothiazolone,
(b) de 0,7 % à 10 % en poids, rapporté au poids de ladite composition de fluide, d'une alcanolamine primaire ou plus,
(c) de 2 % à 30 % en poids, rapporté au poids de ladite composition de fluide, d'une alcanolamine tertiaire ou plus,
(d) un stabilisant ou plus sélectionné dans le groupe constitué d'acide iodique, d'acide périodique, de sels iodate, de sels périodate, d'iodopropynylbutylcarbamate, de mercaptobenzothiazole, et de mélanges de ceux-ci,
dans laquelle le rapport en poids dudit stabilisant à ladite 3-isothiazolone non chlorée va de 0,2/1 à 5/1.

2. Une composition de fluide dilué formée par un procédé comprenant le fait de diluer la composition de fluide de la revendication 1 par un facteur de 10 à 50, ladite composition de fluide dilué convenant en tant que fluide d'usinage des métaux.
